# EUROPEAN PATENT APPLICATION

(11) **EP 3 073 248 A1**
(43) Date of publication of application: **28.09.2016**
(21) Application number: 16161758.4
(22) Date of filing: 22.03.2016
(51) Int. Cl.: G01N 21/17, G01N 29/24, G01N 33/28, G01N 1/22

(54) **TRACE GAS MEASUREMENT APPARATUS FOR ELECTRICAL EQUIPMENT**

(30) Priority: 26.03.2015 US 201514669751
(71) Applicant: General Electric Company, Schenectady, NY 12345 (US)
(72) Inventor: ROBINSON, David Peter, Lisburn, Antrim BT28 2LU (GB)
(74) Representative: Cleary, Fidelma

(57) **Abstract**

Provided is a trace gas measurement apparatus for electrical equipment (50) that includes a sample cell (110) corresponding and connectable to the electrical equipment and comprising a head space (112) and configured to collect an oil sample (114) from the electrical equipment. The trace gas measurement apparatus also includes an analysis module (130) in communication with the sample cell (110), having an analysis chamber that includes a first measuring device (140) at a first side thereof and a second measuring device disposed at a second side thereof at an axis that is substantially perpendicular to a membrane surface of the first measuring device 9140), and configured to measure and analyze trace gases (113) from an oil sample (114) received from the head space (112) within the sample cell (110).

## Description

### TECHNICAL FIELD

The technical field relates generally to trace gas measurement apparatus. In particularly, the present invention relates to trace gas measurement apparatus for measuring and analyzing trace gases in electrical equipment (e.g., a transformer).

### BACKGROUND

Trace gas in electrical equipment is typically generated from electrical insulating oil used in electrical equipment, which generates and distributes electrical power. Some examples of electrical equipment include transformers, tap-changers and circuit breakers. When a fault occurs within the electrical equipment a trace gas (i.e., a fault gas) may be generated in the electrical insulating oil. Therefore, trace gas measurements are used to provide an operational and health status of the electrical equipment.

For example, in a transformer, when faults e.g., arcing and overheating occur, gases such as methane and carbon dioxide or carbon monoxide are present in the insulating oil of the transformer. Measurements of these trace gases can be used to determine the type and the severity of the faults which occur in the electrical equipment. A measurement device such as a photo-acoustic spectroscopy are typically used to obtain trace gas measurements where small vibrations of the molecules in trace gases are generated when subjected to a particular infrared (IR) frequencies of light, however external vibrations of measurement device can interfere with the measurement process.

### SUMMARY OF THE EMBODIMENTS

The various embodiments of the present disclosure are configured to provide trace gas measurement apparatus which distinguishes between internal vibrations of the trace gases and external vibrations of the measurement apparatus, to efficiently measure trace gases in electrical insulating oil of electrical equipment.

In one exemplary embodiment, a trace gas measurement apparatus is provided. The trace gas measurement apparatus includes a sample cell corresponding and connectable to the electrical equipment and comprising a head space and configured to collect an oil sample from the electrical equipment. The trace gas measurement apparatus also includes an analysis module in communication with the sample cell, having an analysis chamber that includes a first measuring device at a first side thereof and a second measuring device disposed at a second side thereof at an axis that is substantially perpendicular to the first measuring device, and configured to measure and analyze trace gases from an oil sample received from the head space within the sample cell.

In one exemplary embodiment, a method is provided. The method includes receiving trace gases within the gas cell of the analysis chamber, from a sample cell in communication with the electrical equipment; applying infrared signals to excite the trace gases and generating pressure waves; detecting amplitudes from the pressure waves using a first measuring device in communication with the gas cell; and detecting signals resulting from external vibrations via a second measuring device positioned along an axis that is substantially perpendicular to the membrane surface of the first measuring device.

In another exemplary embodiment, a method is provided. The method includes receiving trace gases within the trace gas cell of an analysis chamber, from a sample cell in communication with the electrical equipment; detecting signals from a first measuring device disposed at a first side of the analysis chamber and the second measuring device at a second side of the analysis chamber that is substantially perpendicular to the membrane surface of the first side; balancing the signals prior to performing trace gas measurement operation; applying infrared signals to excite the trace gases and generating pressure waves; detecting amplitudes from the pressure waves using a first measuring device in communication with the gas cell; and detecting signals resulting from external vibrations via a second measuring device positioned along an axis that is substantially perpendicular to that of the membrane surface of the first measuring device.

The foregoing has broadly outlined some of the aspects and features of various embodiments, which should be construed to be merely illustrative of various potential applications of the disclosure. Other beneficial results can be obtained by applying the disclosed information in a different manner or by combining various aspects of the disclosed embodiments. Accordingly, other aspects and a more comprehensive understanding may be obtained by referring to the detailed description of the exemplary embodiments taken in conjunction with the accompanying drawings, in addition to the scope defined by the claims.

### DESCRIPTION OF THE DRAWINGS

FIG. 1 is a block diagram illustrating a trace gas measurement apparatus that can be implemented within one or more embodiments of the present invention.
FIG. 2 is a block diagram of an analysis module of the trace gas measurement apparatus shown in FIG. 1 that can be implemented within one or more embodiments of the present invention.
FIG. 3 is a flow diagram illustrating an exemplary method of implementing an embodiment of the present invention.
FIG. 4 is a flow diagram illustrating an exemplary method of implementing an alternative embodiment of the present invention.

The drawings are only for purposes of illustrating preferred embodiments and are not to be construed as limiting the disclosure. Given the following enabling description of the drawings, the novel aspects of the present disclosure should become evident to a person of ordinary skill in the art. This detailed description uses numerical and letter designations to refer to features in the drawings. Like or similar designations in the drawings and description have been used to refer to like or similar parts of embodiments of the invention.

### DETAILED DESCRIPTION OF THE EMBODIMENTS

As required, detailed embodiments are disclosed herein. It must be understood that the disclosed embodiments are merely exemplary of various and alternative forms. As used herein, the word "exemplary" is used expansively to refer to embodiments that serve as illustrations, specimens, models, or patterns. The figures are not necessarily to scale and some features may be exaggerated or minimized to show details of particular components. In other instances, well-known components, systems, materials, or methods that are known to those having ordinary skill in the art have not been described in detail in order to avoid obscuring the present disclosure. Therefore, specific structural and functional details disclosed herein are not to be interpreted as limiting, but merely as a basis for the claims and as a representative basis for teaching one skilled in the art.

Exemplary embodiments of the present invention provides a trace gas measurement apparatus for performing dissolved gas analysis (DGA) on electrical insulating oil flowing within electrical equipment (e.g., transformers, circuit breakers, or tap changers). The trace gas measurement apparatus may be implemented within a portable gas analyzer (PGA). The DGA process is used to determine the health (e.g., the occurrence any faults or failure) of the electrical equipment and the current state of operation thereof. The trace gas measurement apparatus effectively performs the DGA testing by eliminating vibration signals received externally from an analysis module of the trace gas measurement apparatus by employing an accelerometer to detect the external vibrations. The signals obtained from the external vibrations by the accelerometer are cancelled from the signals obtained from the internal vibrations resulting from pressure wave signals received at a microphone of the trace gas measurement apparatus.

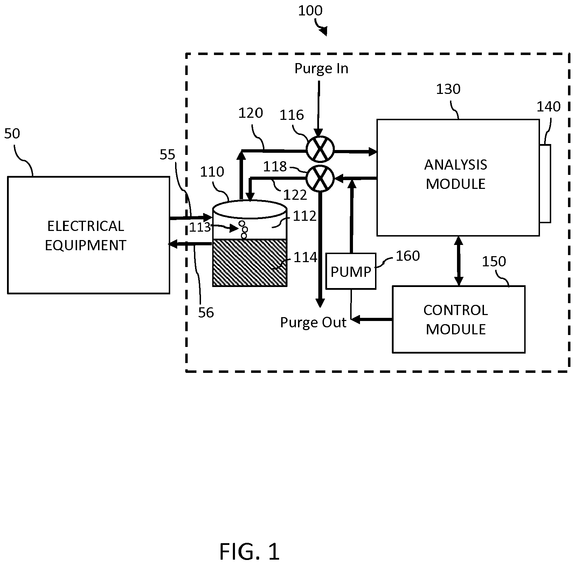

FIG. 1 is a block diagram illustrating a trace gas measurement apparatus that can be implemented within one or more embodiments of the present invention. As shown in FIG. 1, the trace gas measurement apparatus 100 is connectable to and communicates directly with electrical equipment 50. This communication may be performed in real-time, on-line during operation of the electrical equipment 50. The trace gas measurement apparatus 100 may be disposed in direct contact with the electrical equipment 50 or in a remote location while maintaining communication with the electrical equipment 50. The present invention is not limited to the trace gas measurement apparatus 100 being disposed in any particular location, the location may be any location suitable for the purposes set forth herein. Further, the present invention is not limited to the electrical equipment including any particular type or number of electrical equipment components (e.g., transformers, tap changers, and/or circuit breakers), and may vary accordingly.

The trace gas measurement apparatus 100 includes at least one sample cell 110 corresponding to and connectable to the electrical equipment 50, and including a head space 112 and an oil sample 114. The sample cell 110 collects the oil sample 114 of insulating oil flowing through the electrical equipment 50. The trace gas measurement apparatus 100 further includes a plurality of valves 116 and 118 within respective forward and return paths 120 and 122 connecting the sample cell 110 to an analysis module 130 for performing DGA. The present invention is not limited to using any particular type of control mechanism for stopping and starting of flow within the forward and return paths 120 and 122, and may vary accordingly.

According to one or more embodiments, a first measuring device, e.g., at least one microphone 140, is disposed adjacent to the analysis module 130. Any type of transducer or sensor for converting sound into electrical signals may be implement within the present invention suitable for the purpose set forth herein. According to some embodiments, a single microphone 140 is provided, however, the present invention is not limited hereto. The present invention is not limited to use of a microphone, other devices may be employed such as a laser reflected by a thin reflective membrane, a strain gauge attached to a thin membrane, or an inertially small accelerometer attached to a thin membrane.

A control module 150 is also provided in communication with the analysis module 130, and a circulation pump 160 is connected between the control module 150 and the forward and/or return paths 120 and 122.

Further as shown, the oil sample 114 in the sample cell 110 is supplied via a forward line 55 from the electrical equipment 50 to the sample cell 110 during operation of the trace gas measurement apparatus 100. And the oil may be returned to the electrical equipment 50 via the return line 56, if desired. The oil sample 114 resides in the sample cell for a predetermined period of time during which a measurement and analysis operation is to be performed. Although a single sample cell 110 is provided, a plurality of sample cells 110 may be provided to accommodate multiple electrical equipment components as needed. Alternatively, multiple electrical equipment components may be connected to a single sample cell 110.

Further in operation, the oil sample 114 which is drawn from the electrical equipment 50 is agitated by an agitator (not shown) to cause dissolved gases (i.e., trace gases) 113 to be released into the head space 112. When the valve 116 is opened in a first position, the trace gases 113 are transferred via the forward path 120 to the analysis module 130 for performing analysis thereof.

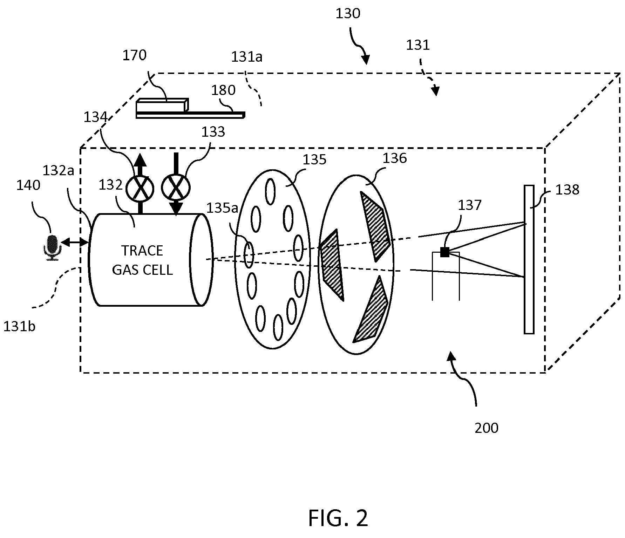

Depending on the measurement operation being implemented, the return valve 118 may be set to return trace gases 113 from the analysis module 130 back to the sample cell 110 (e.g., in a closed loop arrangement) or to cause the trace gases 113 from the analysis module 130 to be purged out of the trace gas measurement apparatus 100 via the valve 118 (e.g., in an open circuit arrangement). Additional details regarding the measurement operation will be discussed below with reference to Fig. 2.

According to one or more embodiments, the control module 150 which includes a microcontroller or microprocessor programmed with computer software for performing analysis of the gases 113 when supplied to the analysis module 130. The control module 150 controls the operation of the analysis module 130 and the circulation pump 160. The control module 150 may be any type of computing device capable of performing the operations of the present invention.

The circulation pump 160 is disposed within the return path 122 and/or forward path 120 for controlling the flow of fluid along the forward and return paths 120 and 122 between the electrical equipment to the analysis module 130. The present invention is not limited to the use of any particular type of pump device, and therefore any pump device suitable for the purposes set forth herein may be employed.

FIG. 2 is a block diagram of the analysis module 130 of the trace gas measurement apparatus 100. As shown in FIG. 2, the analysis module 130 comprises an analysis chamber 131 housing all of the components of the analysis module 130. The analysis chamber 131 includes a trace gas cell 132, having input and output lines and valves 133 and 134 for controlling the flow of trace gases 113 into the trace gas cell 132 when desired. The analysis chamber 131 further includes a photo-acoustic spectrometer 200 comprising a filter selector 135, a strobe wheel 136, a broadband IR frequency source 137 and a reflector 138. The microphone 140 is disposed at a surface of the analysis chamber 131 and in communication with the trace gas cell 132 (as indicated by the arrow).

According to an embodiment of the present invention, the analysis module 130 further comprises a second measuring device 170, e.g., an accelerometer 170, mounted either directly on a surface 131a of the analysis chamber 131 via mounting components (e.g., bolts), at a manifold of the analysis module 130, or mounted indirectly to the surface 131a of the analysis chamber 131 via another component (i.e., a printed circuit board (PCB) 180) mounted to the surface. The accelerometer 170 is aligned so that the axis thereof is detecting acceleration.

According to one embodiment, the accelerometer 170 is aligned so that the axis its detecting is on the same axis as that which the microphone 140 is picking up signals. According to embodiments, the accelerometer 170 includes its main axis aligned with the deflection of the microphone's 140 membrane, adding on more detection axes onto the accelerometer 170 allows for less critical placement of the primary accelerometer axis.

During the measurement operation, the photo-acoustic spectrometer 200 performs infrared (IR) photo-acoustic spectroscopy. Within the spectrometer 200, the broadband IR source 137 supplies IR light to be reflected via the reflector 138 in a direction trace gas cell 132. The strobe wheel 136 directs the light reflected to pass through a sequence of optical filters 135a of the optical filter selector 135. Each optical filter 135a is arranged to pass IR light in a respective frequency band associated with a particular target trace gas 113 (e.g., methane), to direct radiation into the trace gas cell 132, via window thereof, which contains a sample of trace gas 113 to be analyzed.

Each target trace gas 113 within the trace gas cell 132 would then absorb energy at its respective resonant frequency, causing a vibration/rotation in the molecules of the target trace gas 113. The absorbed energy is then released creating pressure waves. The trace gas cell 132 is formed of a cylindrical shape in a vertical direction, for example however it is not limited hereto and may vary according. The microphone 140 is disposed on a side 132a of the analysis module 130 to be in communication with the trace gas cell 132 at a side 132a thereof.

The gas cell 132 is connected to the microphone 140 such that when the trace gases 113 in the gas cell 132 are contracting and expanding, pressure waves therefrom are directed towards the microphone 140. The microphone 140 detects the pressure waves and the amplitudes thereof are used to determine the quantity of the target trace gases 113.

According to one or more embodiments, the accelerometer 170 is disposed at location on a side 131a of the analysis chamber 131. The axis of the accelerometer 170 is perpendicular to an axis of the microphone 140 located at another side 131b of the analysis chamber 131.

The accelerometer 170 is able to detect and monitor external vibrations external to the analysis module 130. These external vibrations may interfere with the measurements of the internal vibrations of the pressure waves generated within the trace gas cell 132 during the measurement process. To avoid the interference, embodiments of the present invention employ the accelerometer 170. The accelerometer 170 continuously monitors and detects the external vibrations and converts them to electrical signals to be subtracted from the electrical signals detected by the microphone 140, to thereby determine the actual measurement for the target gases as desired. The measurements are used to determine the health of the electrical equipment 50.

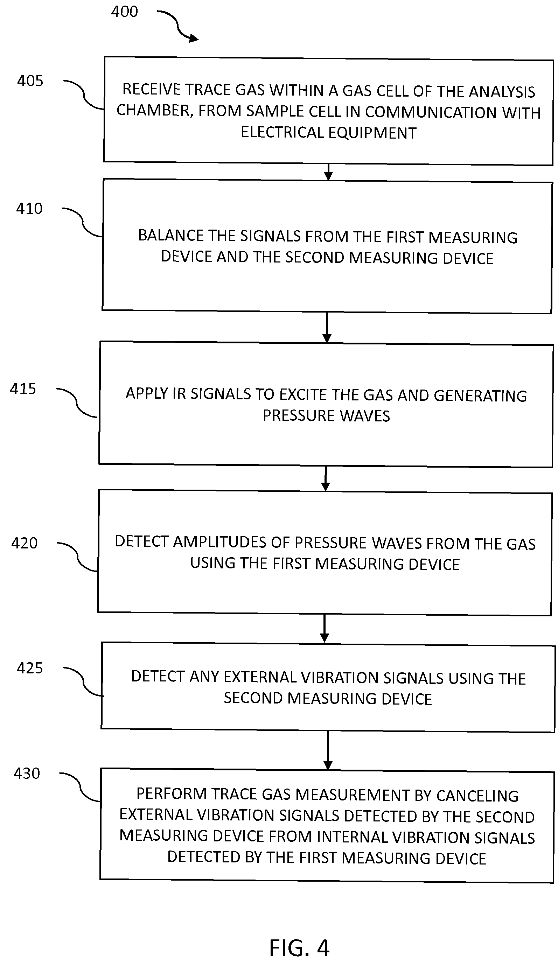

Further, the data associated with the external vibrations as detected by the accelerometer 170 may be used to determine any electrical components which create interference, e.g., noise during the measurement operation, and to perform adjustments of the electrical components as necessary to eliminate the interference. The present invention is not limited to the above-mentioned measurement method. A measurement method according to other embodiments as illustrated in Fig. 4 and discussed below may also be implemented. The accelerometer 170 may be a 1-axis type mounted at a location perpendicular to the axis of the microphone 140 as shown in FIG. 2 and discussed above, to prevent external interference with the internal vibrations resulting from the pressure waves detection performed by the microphone 140.

Alternatively, the accelerometer 170 is not limited to any particular type, and may be of a 2-axis or 3-axis type or any other type of accelerometer which is suitable for the purposes set forth herein. Thus, the accelerometer 170 is not limited to being disposed in any particular location along the analysis chamber 131. A 3-axis accelerometer 170 is able to resolve and work out the vibration experienced by the microphone's 140 membrane.

After measurements are taken, valves 116 and 118 are open for performing a flushing or purging period under the control of the control module 150, to thereby obtain a new trace gas sample (i.e., trace gases 113). The analysis module 130 comprises an inlet valve 133 for isolating it from the forward flow path 120 and an outlet valve 134 for isolating it from the return flow path 122. The valves 133 and 134 are open to allow the contents in the analysis module 130 to be flushed out by gases or liquid, such as clean air or purging fluid received via valve 116 from the "Purge In" flow path, flow through the gas cell 132 and out at the "Purge Out" flow path in the open circuit arrangement.

After the flushing period, the inlet and outlet valves 133 and 134 are closed and the trace gas cell 132 may receive the new gas sample including trace gases 113 for analysis.

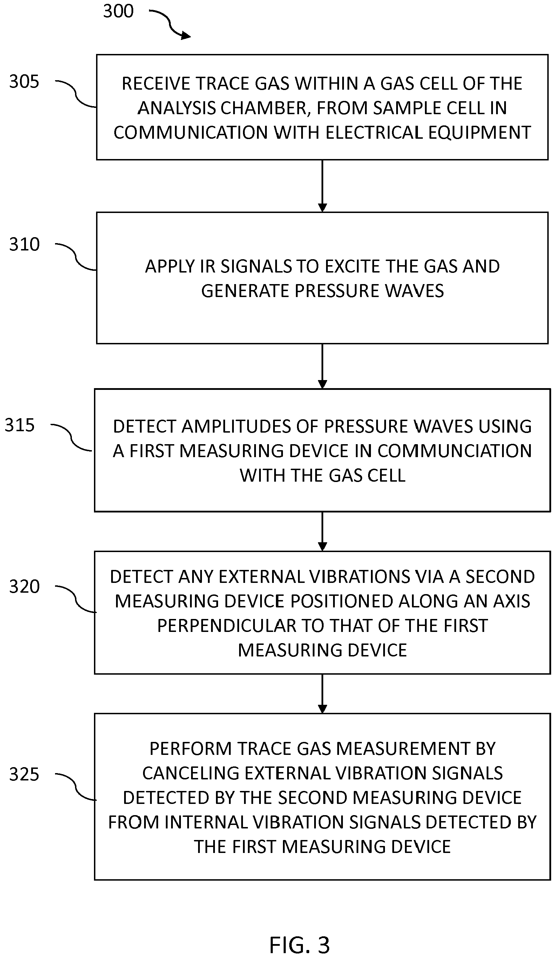

Trace gas measurement methods performed in the analysis module 130 in accordance with embodiments of the present invention will now be discussed with reference to FIGS. 3 and 4. FIG. 3 is a flow diagram illustrating an exemplary method 300 of implementing an embodiment of the present invention. FIG. 4 is a flow diagram illustrating an exemplary method 400 of implementing an alternative embodiment of the present invention.

As shown in FIG. 3, at operation 305 in method 300, trace gases are received within the gas cell of the analysis chamber, from the sample cell in communication with the electrical equipment.

From operation 305, the process continues to operation 310 where IR signals are applied to excite the trace gases and generate pressure waves. At operation 315, amplitudes from the pressure waves are detected using a first measuring device (e.g., a microphone) in communication with the gas cell.

From operation 315, the process continues to operation 320 where signals resulting from external vibrations are detected via a second measuring device (e.g., an accelerometer) positioned along an axis that is substantially perpendicular to that of the first measuring device.

At operation 325, trace gas measurements are then performed by cancelling (i.e., subtracting) the signals from the external vibrations as detected by the second measuring device from the signals resulting from the internal vibrations as detected by the first measuring device.

FIG. 4 is a flow diagram illustrating an exemplary method 400 of implementing an alternative embodiment of the present invention. As shown in method 400, at operation 405, trace gases are received within the trace gas cell of the analysis chamber, from the sample cell in communication with the electrical equipment.

From operation 405, the process continues to operation 410 where signals from the first measuring device (i.e, the microphone) and the second measuring device (i.e., the accelerometer) are balanced prior to performing the measuring operation. The balancing operation may be performed by applying a gain, or multiplying via a multiplier, to the signal of the accelerometer so that when the trace gas cell is agitated and not excited, the two signals can be balanced. Thus, when the signals resulting from the internal vibrations are generated only the microphone responds thereto and not the accelerometer. However, in the event of external vibration, the accelerometer will read the external vibration and cancel it from the signals received at the microphone. Data collection of the accelerometer signal may be added for further assessment of the location of the external vibration.

From operation 410, the process continues to operation 415, where infrared (IR) signals are applied to the trace gases within the gas cell. From operation 415, the process continues to operation 420 where amplitudes from pressure waves in the gas cell are detected using the first measuring device (i.e., the microphone) in communication with the gas cell.

From operation 420, the process continues to operation 425 where signals resulting from external vibrations are detected via the second measuring device (i.e., the accelerometer) positioned along an axis that is substantially perpendicular to the first measuring device's membrane surface. Then at operation 430, trace gas measurements are then performed by cancelling (i.e., subtracting) the signals from the external vibrations as detected by the second measuring device from the signals resulting from the internal vibrations as detected by the first measuring device.

The measurement apparatus of the present invention may be used in an on line measurement type arrangement with electrical equipment such as a main transformer and/or tank changer. The measurement apparatus may further be implemented in real-time to determine the condition of the total electrical system (e.g., a transformer system). These faults can be detected early, to minimize cost associated with unplanned outages and any electrical equipment failure.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A trace gas measurement apparatus (100) for electrical equipment (50), the trace gas measurement apparatus (100) comprising:
at least one sample cell (110) (i) connectable to the electrical equipment (50) and including a head space (112) and (ii) configured to collect an oil sample (114) from the electrical equipment (50); and
an analysis module (130) in communication with the at least one sample cell (110), and including an analysis chamber (131) having a first measuring device (140) at a first side (132a) thereof and a second measuring device (170) disposed at a second side (131a) thereof at an axis substantially perpendicular to a membrane surface of the first measuring device (140);
wherein the analysis module (130) is configured to measure and analyze trace gases (113) from the oil sample (114), as received from the head space (112) within the sample cell (110).

2. The trace gas measurement apparatus of claim 1, further comprising:
a control module (150) configured to control an operation of the analysis module (130).

3. The trace gas measurement apparatus of claim 2, further comprising:
a circulation pump (160) configured to control flow of the trace gases (113) between the at least one sample cell (110) and the analysis module (130), wherein the control module (150) is further configured to control the circulation pump (160).

4. The trace gas measurement apparatus of claim 3, wherein the analysis chamber (131) further comprises:
a trace gas cell (132) in communication with the first measuring device (140) and receiving trace gases (113) from the sample cell (110); and
a device (135a) configured to supply infrared light in the direction of the trace gas cell (132), wherein the trace gases (113) absorb energy at respective resonant frequency, causing internal vibrations in molecules of the trace gases (113) within the trace gas cell (132), and wherein the first measuring device (140) is configured to detect amplitudes resulting from pressure waves of the internal vibrations.

5. The trace gas measurement apparatus of claim 4, wherein the first measuring device (140) comprises a single microphone, thin membrane measured with a laser beam or an accelerometer, or other measuring device.

6. The trace gas measurement apparatus of claim 5, wherein the second measuring device (170) is disposed at another side (131a) of the analysis chamber (131) substantially perpendicular to a location of the first measuring device (140) at the side (132a) of the analysis chamber (131), and configured to continuously detect and monitor external vibrations external to the analysis chamber (131).

7. The trace gas measurement apparatus of any preceding claim, wherein the second measuring device (170) comprises an accelerometer.

8. The trace gas measurement apparatus of claim 7, wherein the control module (150) is configured to perform a measurement operation by cancelling signals as detected by the second measuring device (170) from signals as detected by the first measuring device.

9. The trace gas measurement apparatus of claim 8, wherein the control module (150) is configured to determine the health of the electrical equipment (50) based on the signals detected by the first measuring device (140).

10. A trace gas measurement method to be performed on electrical equipment (50), comprising:
receiving trace gases (113) within a trace gas cell (132) of an analysis chamber (132), from a sample cell (110) in communication with the electrical equipment (50);
applying infrared signals to excite the trace gases (113) and generating pressure waves;
detecting amplitudes from the pressure waves using a first measuring device (140) in communication with the gas cell (132); and
detecting signals resulting from external vibrations via a second measuring device (170) positioned along an axis substantially perpendicular to a membrane surface of the first measuring device (140).

11. The method of claim 10, wherein the first measuring device (140) comprises a single microphone, thin membrane measured with a laser beam or an accelerometer, or other measuring device.

12. The method of claim 10 or 11, wherein the second measuring device (170) comprises an accelerometer.

13. The method of any of claims 10 to 12, further comprising:
cancelling the signals from the external vibrations as detected by the second measuring device (170) from the signals resulting from the internal vibrations as detected by the first measuring device (140).

14. The method of any of claims 10 to 13, further comprising:
detecting signals from the first measuring device (140) disposed at a first side (131a) of the analysis chamber (132) and the second measuring device (170) at a second side (132a) of the analysis chamber (132) perpendicular to the first side (131a; and
balancing the signals prior to performing trace gas measurement operation.

15. The method of claim 14, wherein balancing is performed by applying a gain, or multiplying via a multiplier, to the signal of the second measuring device (170).
